# EUROPEAN PATENT SPECIFICATION

(11) **EP 3 428 169 B1**
(45) Date of publication and mention of the grant of the patent: **20.01.2021**
(21) Application number: 18183102.5
(22) Date of filing: 12.07.2018
(51) Int. Cl.: C07D 493/04, A61P 35/00, A61K 31/34

(54) **6,6A-DIHYDRO FURO[3,2-B]FURAN-2-(5H)ONE DERIVATIVES, THEIR PREPARATION AND USE FOR TREATING TUMORS**
6,6A-DIHYDRO FURO[3,2-B]FURAN-2-(5H)ON DERIVATE, IHRE HERSTELLUNG UND VERWENDUNG ZUR BEHANDLUNG VON KREBS
DERIVÉS DE 6,6A-DIHYDRO FURO[3,2-B]FURAN-2-(5H)ONE, LEUR PRÉPARATION ET UTILISATION POUR LE TRAITEMENT DES TUMEURS

(30) Priority: 13.07.2017 IT 201700078586
(43) Date of publication of application: 16.01.2019
(73) Proprietor: Universita' Della Calabria, 87036 Rende, Cosenza (IT)
(72) Inventor: GABRIELE, Bartolo, 87036 RENDE, COSENZA (IT); CHIMENTO, Adele, 87036 RENDE, CONSENZA (IT); MANCUSO, Raffaella, 87036 RENDE, COSENZA (IT); PEZZI, Vincenzo, 87036 RENDE, COSENZA (IT); ZICCARELLI, Ida, 87036 RENDE, COSENZA (IT); SIRIANNI, Rosa, 87036 RENDE, COSENZA (IT)
(74) Representative: Biggi, Cristina

(56) References cited:
- CN-B- 103 655 552
- Alberto Bertucco ET AL: "Michael and substitution reactions of bicyclic tetronic, tetramic and thiotetronic esters", Tetrahedron, 1 January 1994 (1994-01-01), pages 8237-8252, XP055462160, DOI: 10.1016/S0040-4020(01)85305-9 Retrieved from the Internet: URL:https://ac.els-cdn.com/S00404020018530 59/1-s2.0-S0040402001853059-main.pdf?_tid= deaa1d4f-6f92-44df-bb96-e9b4a32d26ec&acdna t=1521734938_91c0695f91ee8816bd28d59868086 26c
- BITTNER ET AL: "A synthetic approach to the plakortones", TETRAHEDRON LETTERS, ELSEVIER, AMSTERDAM, NL, vol. 40, no. 17, 23 April 1999 (1999-04-23), pages 3455-3456, XP022502882, ISSN: 0040-4039, DOI: 10.1016/S0040-4039(99)00421-9
- ZHANG QIURONG ET AL: "Synthesis and biological evaluation of 2,6-dichloropurine bicyclonucleosides containing a triazolyl-carbohydrate moiety", CARBOHYDRATE RESEARCH, vol. 382, 2013, pages 65-70, XP028780511, ISSN: 0008-6215, DOI: 10.1016/J.CARRES.2013.10.004
- BENOIT BORDIGNON ET AL: "Ascorbic acid derivatives as a new class of antiproliferative molecules", CANCER LETTERS, vol. 338, no. 2, 1 September 2013 (2013-09-01), pages 317-327, XP055462140, US ISSN: 0304-3835, DOI: 10.1016/j.canlet.2013.06.015

## Description

### TECHNICAL FIELD

The present invention relates to 6,6*a*-dihydrofuro[3,2-*b*]furan-2-(5*H*)one derivatives and the use thereof as antitumour agents.

### PRIOR ART

Pharmacological research in the field of the development of new antitumour agents is of primary importance today.

Breast cancer (also called breast tumour) represents the main cause of death due to cancer in women around the world, above all in industrialised countries. Genetic factors (for example mutations in the tumour-suppressor genes BRCA1 and BRCA2), endocrine factors (exposure to oestrogens) and lifestyle (diet) can be involved in the onset of this disease. The expression of specific molecular markers, or receptors used by tumour cells to activate proliferative mechanisms, has led breast cancer to be distinguished into various subtypes: firstly, breast cancers are divided into "HR positive" (Hormone Receptors, HR+) tumours and "HR negative" tumours. HR positive tumours include, in turn, (i) oestrogen-receptor positive tumours (ER+), (ii) progestin-receptor positive tumours (PR+) and (iii) human epidermal growth factor receptor 2-positive tumours (HER2+). HR negative tumours are defined as "triple negative" breast tumours (TNBCs), since they do not exhibit any of the three types of receptors. Based on the specific characteristics of the tumour forms, the therapeutic approaches are different. Conventional chemotherapy aims to block the proliferation of tumour cells, but does not discriminate them from normal (non-tumour) cells undergoing rapid division (indiscriminate targeting). Hormone therapy (or "endocrine therapy") employs drugs effective in blocking the growth of tumour cells which express oestrogen receptors (ERs) and/or progestin receptors (PRs). Another promising therapeutic approach is based on so-called "molecular targeted therapy", which uses drugs (for example, monoclonal antibodies targeted against growth factors and their receptors, growth factor receptor inhibitors, vaccines) capable of interfering with different molecules, both those present on the cell surface and intracellular ones, by inactivating proteins involved, in turn, in tumour growth. For example, in therapy for HER2+ tumours, use is made of the monoclonal antibody trastuzumab or the inhibitor lapatinib.

Unfortunately, despite the major steps forward made in respect of early diagnosis and therapeutic treatments for breast cancer, many patients manifest a relapse after an initial positive response to treatment with conventional chemotherapeutics or endocrine therapy. Even though the five-year survival rate for patients with early-stage breast cancer who receive treatment is extremely high, some patients with breast cancer in an advanced stage can suffer a relapse within 10 years of treatment. It is estimated overall that about 11% of women with breast cancer can have a recurrence five years after the end of treatment, and about 20% after 10 years. The recurrence rate for HR positive tumours is lower (5-year recurrence rate of about 8%) than the recurrence rate for the tumour subtypes that overexpress HER2 or have a triple negative phenotype, for which the 5-year recurrence rate is 15-20%. "Triple negative" tumours (TNBCs), which represent only 15-20% of all types of breast tumours, are the most difficult forms to treat, since they are commonly associated with an increased risk of metastasis and are susceptible neither to endocrine therapy (which uses anti-oestrogen drugs such as tamoxifen or aromatase inhibitors in ER+ tumours) nor targeted therapy for HER2+ tumours. Therefore, it is of great importance to continue to explore new therapeutic opportunities offered by the discovery of new effective agents with antitumour activity, which can become effective drugs for the treatment of different breast tumour subtypes. In particular, it is necessary to identify inexpensive drugs capable of acting on several tumour subtypes and of inhibiting the growth not only of tumour cells which express the receptors involved in tumour progression (ER, PR, HER2), but also of cells lacking such receptors, and which manifest a more aggressive phenotype (tumours of the "triple negative" type).

The present invention thus has the main task of providing new compounds with a therapeutic action and which have application in the medical field, in particular in the treatment of tumours. Within the scope of this task, one object of the invention is to provide compounds effective in the treatment of breast tumours, and in particular in the treatment of breast tumours of triple negative type (TNBCs).

Another object of the invention is to provide compounds that are selective for tumour cells, in such a way as to reduce global toxicity in the body (and thus side effects) when they are administered to a patient.

The present invention also has the object of providing a process for preparing these new compounds whose execution is simple and rapid and entails limited costs.

### SUMMARY OF THE INVENTION

The present invention relates to compounds having the following formula of structure (A): 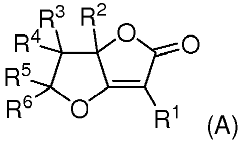 wherein:
R¹ is selected from the group consisting of: H, alkyl, aryl, heteroaryl and trimethylsilyl;
R², R³, R⁴, R⁵ and R⁶ are independently selected from the group consisting of: H, alkyl, aryl and heteroaryl;
and pharmaceutically acceptable salts thereof,
for use as a medicament, in particular for the treatment of tumours.

The invention also relates to the compounds of formula (A) where R¹, R², R³, R⁴, R⁵ and R⁶ are defined as above, wherein when R¹ is methyl or hydrogen, R² is selected from the group consisting of: aryl and heteroaryl.

Furthermore, the invention relates to a process for preparing the compounds of formula (A), wherein a 4-in-1,3-diol compound having the structure (I) 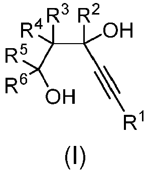 wherein
R¹ is selected from the group consisting of: H, alkyl, aryl, heteroaryl and trimethylsilyl;
R², R³, R⁴, R⁵ and R⁶ are independently selected from the group consisting of: H, alkyl, aryl and heteroaryl,
is reacted with carbon monoxide (CO) and oxygen (O₂) in the presence of a palladium halide (PdX₂) as a catalyst and potassium iodide (KI) as an additive, using a C1-C4 alkyl alcohol as a solvent.

The process consists in a catalytic double cyclisation reaction of the starting substrates (the 4-in-1,3-diol compounds of formula (I)) with the incorporation of a molecule of carbon monoxide, under oxidative conditions. The reaction takes place in a single stage and leads to the obtainment of the 6,6*a*-dihydrofuro[3,2-*b*]furan-2-(5*H*)one bicyclic derivatives of formula (A) and water as products.

### DESCRIPTION OF THE FIGURES

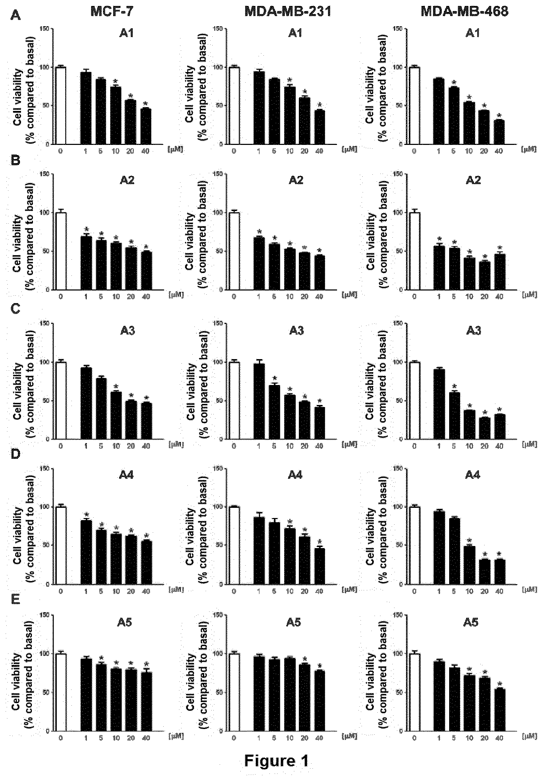

Figure 1 shows graphs relating to the effect of some 6,6*a*-dihydrofuro[3,2-*b*]furan-2-(5H)one derivatives of formula (A) on the viability of MCF-7 (ER+, PR+, HER2-), MDA-MB-231 (ER-, PR-, HER2-) and MDA-MB-468 (ER-, PR-, HER2-) tumour cells. The cells were treated with different concentrations (1 µM, 5 µM, 10 µM, 20 µM, 40 µM) of compounds A1 (figure 1A), A2 (figure 1B), A3 (figure 1C), A4 (figure 1D) and A5 (figure 1E) for 72 h; the control cells were not treated (0 µM, basal). Cell viability was evaluated with the MTT assay. The results are expressed as mean values ± the standard deviation (SD) of three independent experiments, each one conducted in triplicate; the statistical difference between the control (basal, not treated) and treated samples was evaluated using GraphPad Prism 5.0 software (GraphPad Software, Inc; La Jolla, CA). The comparison among several groups was performed by analysis of variance (ANOVA) using the Bonferroni or Dunn's test as the post test. Values of p <0.05 were considered significant relative to the basal value and indicated with an asterisk (*).

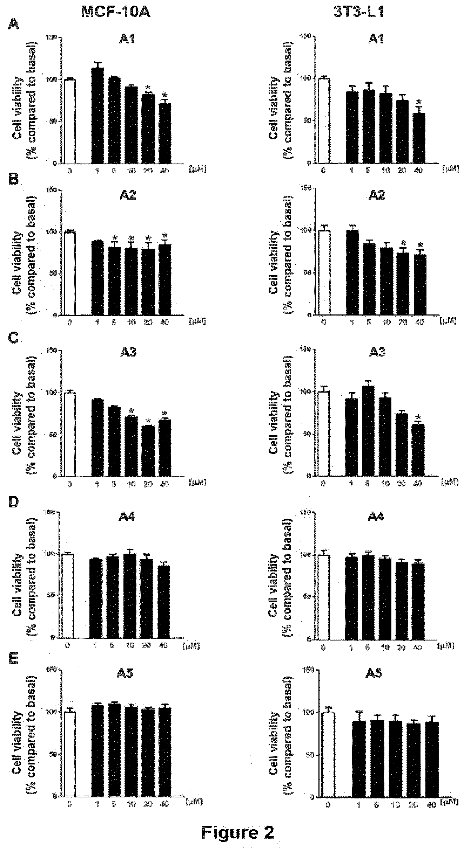

Figure 2 shows graphs relating to the effect of some 6,6*a*-dihydrofuro[3,2-*b*]furan-2-(5H)one derivatives of formula (A) on the viability non-tumour cells: MCF-10A (immortalised human mammary epithelium non-tumour cells) and 3T3-L1 (immortalised mouse fibroblasts). The cells were treated with different concentrations (1 µM, 5 µM, 10 µM, 20 µM, 40 µM) of compounds A1 (figure 1A), A2 (figure 1B), A3 (figure 1C), A4 (figure 1D) and A5 (figure 1E) for 72 h; the control cells were not treated (0 µM, basal). Cell viability was evaluated with the MTT assay. The results are expressed as mean values ± the standard deviation (SD) of three independent experiments, each one conducted in triplicate; the statistical difference between the control (basal, not treated) and treated samples was evaluated using GraphPad Prism 5.0 software (GraphPad Software, Inc; La Jolla, CA). The comparison among several groups was performed by analysis of variance (ANOVA) using the Bonferroni or Dunn's test as the post test. Values of p <0.05 were considered significant relative to the basal value and indicated with an asterisk (*).

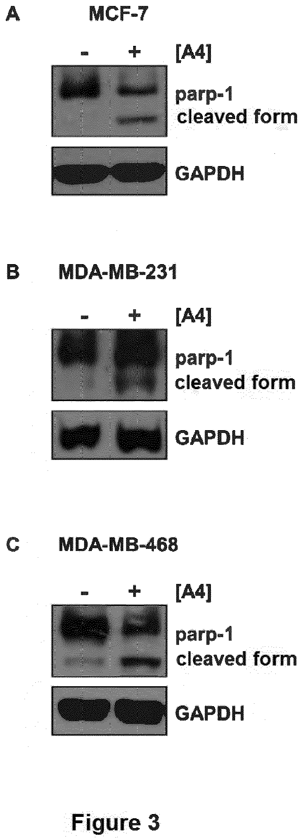

Figure 3 (A, B, C) shows the cleavage of the parp-1 protein in the MCF-7, MDA-MB-231 and MDA-MB-468 cells following treatment with compound A4. The effects on the cleavage of the parp-1 protein in the MCF-7 (figure 3A), MDA-MB-231 (figure 3B) and MDA-MB-468 (figure 3C) cells were evaluated by western blot analysis. The analysis was performed on equal amounts of total proteins. The GAPDH protein was used as a loading control. Each western blot analysis was repeated three times, in an independent manner, and comparable results were obtained; the images in figure 3, which show the results of one of the three experiments, are thus representative of the analysis.

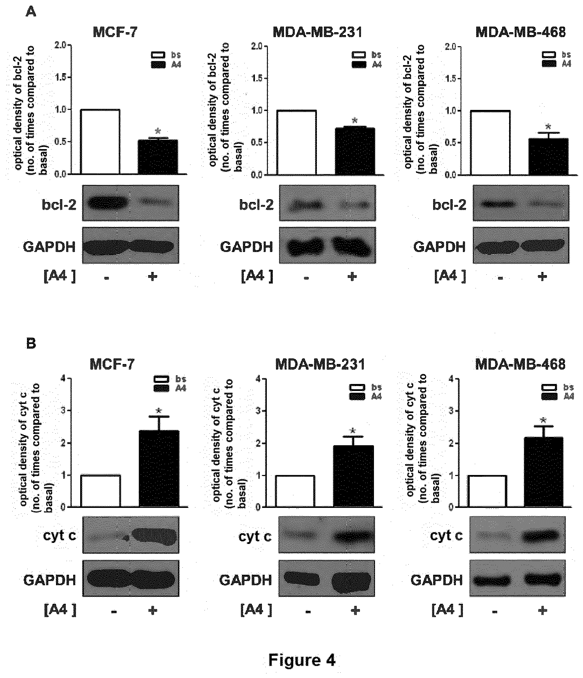

Figure 4 shows the western blot analysis images related to the effects of the treatment with compound A4 on the expression of the apoptotic markers bcl-2 and cytochrome c present in the cytosolic protein fraction, in the MCF-7, MDA-MB-231 and MDA-MB-468 cells. A densitometric band analysis was performed on the western blot images, and the respective values are shown in the graphs above the images themselves.

More precisely, the MCF-7, MDA-MB-231 and MDA-MB-468 cells were not treated (-) or treated (+) with A4 (20 µM) for 48h. The western blot analysis of bcl-2 (figure 4A) and cytochrome c in the cytosolic protein fraction (figure 4B) was performed on equal amounts of proteins. The GAPDH protein was used as a loading control. Each western blot analysis was repeated three times, in an independent manner, and comparable results were obtained; the images in figure 4, which show the results of one of the three experiments, are thus representative of the analysis. A densitometric band analysis was performed on the three independent western blot analyses which gave similar results. The results are expressed as mean values ± the standard deviation (SD); the statistical difference between the control (basal, not treated) and the treated sample was evaluated using GraphPad Prism 5.0 software (GraphPad Software, Inc; La Jolla, CA). The comparison between the two groups was performed with Student's t test. Values of p <0.05 were considered significant relative to the basal value and indicated with an asterisk (*).

### DETAILED DESCRIPTION OF THE INVENTION

In the context of the present invention, the term "alkyl" means a linear or branched hydrocarbon radical, preferably containing 1 to 6 carbon atoms, more preferably from 1 to 3 carbon atoms. Examples of alkyls comprise, but are not limited to: methyl, ethyl, *n*-propyl, iso-propyl, *n*-butyl, *sec-*butyl, *iso*-butyl, *tert-butyl, n*-pentyl, *n*-hexyl.
"Aryl" means a monocyclic or polycyclic aromatic radical, preferably monocyclic, derived from an aromatic hydrocarbon from which an atom of hydrogen directly bound to an aromatic ring was removed; examples of aryls comprise, but are not limited to: phenyl and naphthyl.
"Heteroaryl" means a monocyclic or polycyclic aromatic radical, preferably monocyclic, derived from an aromatic hydrocarbon from which an atom of hydrogen directly bound to an aromatic ring was removed, wherein one or more carbon atoms are substituted by one or more heteroatoms such as N, S, and/or O; preferably, a single carbon atom is substituted by a heteroatom. Examples of heteroaryls comprise, but are not limited to: furan, thiophene, pyrrole, pyridine, imidazole, indole, benzimidazole.

A first aspect of the invention relates to compounds having a formula of structure (A) 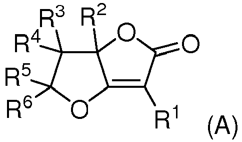 wherein:
R¹ is selected from the group consisting of: H, alkyl, aryl, heteroaryl and trimethylsilyl;
R², R³, R⁴, R⁵ and R⁶ are independently selected from the group consisting of: H, alkyl, aryl and heteroaryl;
and pharmaceutically acceptable salts thereof,
for use as a medication.

Chemically, these compounds are 6,6*a*- dihydrofuro[3,2-*b*]furan-2-(5H)one derivatives.

In one embodiment of the compounds for use of formula (A), R¹, R², R³, R⁴, R⁵ and R⁶ are defined as above and, when R¹ is methyl or hydrogen, R² is selected from the group consisting of: alkyl, aryl and heteroaryl.

In one embodiment of the compounds for use of formula (A), R² can be selected from the group consisting of alkyl, aryl and heteroaryl. R² can preferably be selected from the group consisting of alkyl and aryl. In a particularly preferred embodiment, R² can be methyl. In another particularly preferred embodiment, R² can be phenyl.

In one embodiment of the compounds for use of formula (A), R¹ can be selected from the group consisting of H, aryl and trimethylsilyl; R¹ can preferably be selected from the group consisting of aryl and trimethylsilyl; when R¹ is aryl, it can preferably be phenyl. Furthermore, in a particularly preferred embodiment, R¹ can be trimethylsilyl.

R³ and R⁴ can have the same meaning or different meanings. In one embodiment of the compounds for use of formula (A), R³ and R⁴ can be, independently of each other, hydrogen atoms; R³ and R⁴ can both be hydrogen atoms.

R⁵ and R⁶ can have the same meaning or different meanings. In one embodiment of the compounds for use of formula (I), R⁵ and R⁶ can be selected, independently of each other, from the group consisting of H and alkyl. When R⁵ and R⁶ are independently selected from alkyls, they can preferably be methyl. In a preferred embodiment, R⁵ and R⁶ can both be hydrogen atoms. In another preferred embodiment, R⁵ and R⁶ can both be methyl.

In one embodiment of the compounds for use of formula (A), R¹ can be trimethylsilyl and R⁵ and R⁶ can be independently selected from the group consisting of H and alkyl. R¹ can preferably be trimethylsilyl and R⁵ and R⁶ can both be H; or, preferably, R¹ can be trimethylsilyl and R⁵ and R⁶ can both be alkyl, more preferably methyl.

In one embodiment of the compounds for use of formula (A), R¹ can be aryl, preferably phenyl, and R⁵ and R⁶ can be independently selected from the group consisting of H and alkyl. R¹ can preferably be aryl and R⁵ and R⁶ can both be H; or, preferably, R¹ can be aryl and R⁵ and R⁶ can both be alkyl, more preferably methyl.

The compounds for use according to the invention can for example be selected from the group consisting of (Me = methyl; Ph = phenyl; SiMe₃ = trimethylsilyl): 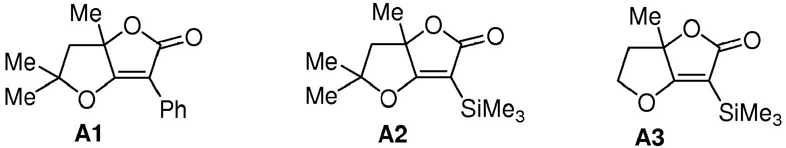 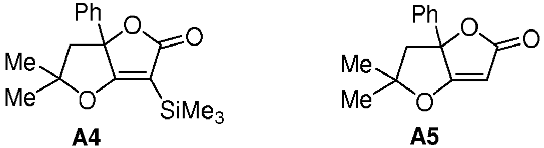 and pharmaceutically acceptable salts thereof.

The compounds for use according to the invention can preferably be selected from the group consisting of: 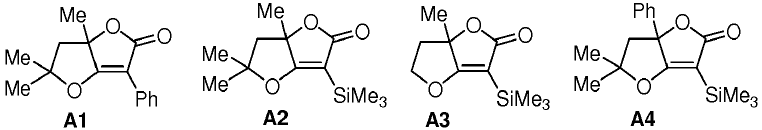 and pharmaceutically acceptable salts thereof.

The above-described 6,6*a*-dihydrofuro[3,2-*b*]furan-2-(5H)one derivatives of formula (A) for use as a medicament, or the pharmaceutically acceptable salts thereof, can be used for the treatment of tumours; they can preferably be used for the treatment of breast tumours. In particular, in one embodiment, the above-described 6,6*a*-dihydrofuro[3,2-*b*]furan-2-(5H)one derivatives of formula (A) for use as a medicament, or the pharmaceutically acceptable salts thereof, can be used for the treatment of HR positive breast tumours, preferably of ER+ and PR+ breast tumours. In another embodiment, the above-described 6,6*a*-dihydrofuro[3,2-*b*]furan-2-(5H)one derivatives of formula (A) for use as a medicament, or the pharmaceutically acceptable salts thereof, can be used for the treatment of breast tumours of the triple negative type, which, as previously explained, have receptors neither for oestrogens, nor for progesterone, nor for the epidermal growth factor, and represent the most aggressive form of breast cancer.

In one embodiment, the compounds of formula (A) for use in the treatment of tumours, and in particular of breast tumours, can be used in addition to or in combination with other already known therapeutic approaches, such as other pharmacologically active substances and/or radiotherapy. The compounds of formula (A) can preferably be used as a medicament for the treatment of human beings.

The antitumour effectiveness of some compounds of the invention of formula (A) was evaluated by means of *in vitro* tests conducted on three breast tumour cell lines: MCF-7, MDA-MB-231 and MDA-MB-468, known in the prior art and commonly used as tumour models. The MCF-7 tumour cell line is used as an experimental model for studying drugs to be used in hormone therapy. The MDA-MB-231 and MDA-MB-468 cell lines are used as models for experimenting with drugs to be used as chemotherapeutic agents or in the target therapy. The tested compounds are the following:

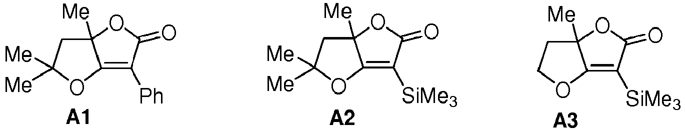

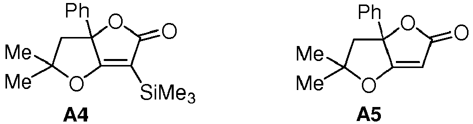

With reference to the examples provided further below and figure 1 appended to the present application, in the tests performed it was observed that the examined compounds, administered in concentrations comprised between 1 µM and 40 µM, brought about a significant inhibition *in vitro* of the viability of the MCF-7, MDA-MB-231 and MDA-MB-468 cell lines. In particular, it was surprisingly observed that the examined compounds are capable of considerably reducing the viability of MDA-MB-468, one of the two cell lines of a triple negative type used as an experimental model for the aggressive forms of cancer. The best results in this respect were obtained with the compound of formula A4. As is known in the art, the inhibition of cell viability is a particularly interesting result in the development of molecules with an antitumour action, since it is directly linked to the inhibition of the proliferation of the cells themselves. Advantageously, the inhibition of cell viability showed to be selective, or in any case less effective, vis-à-vis the tumour cells, as demonstrated by the results for the non-tumour control cell lines MCF-1 0A (immortalised human mammary epithelium non-tumour cells) and 3T3-L1 (immortalised mouse fibroblasts) shown in figure 2. In fact, at the concentrations examined, the viability of MCF-10A and 3T3-L1 cells shows to be slightly reduced (compounds A1, A2, A3) or even not reduced at all (compounds A4 and A5).

One of the main objectives of antitumour therapies is to promote the death of tumour cells, for example by inducing apoptosis of the cells themselves. The compounds of the invention are capable of initiating the death by apoptosis of MCF-7, MDA-MB-231 and MDA-MB-468 tumour cells; also as regards apoptosis, the most satisfactory results were obtained with compound A4. As is known, a group of proteins belonging to the Bcl-2 family intervenes in the regulation of apoptosis; it comprises both pro-apoptotic proteins (such as Bax, Bad, Bak, Bid), and anti-apoptotic proteins (such as Bcl-2 and Bcl-xl), which modulate the cell death execution pathway.

Furthermore, it is also known that proteins of the Bcl-2 family control mitochondrial permeability and the release of cytochrome c from the mitochondria into the cytosol of the cell; the release of cytochrome c into the cytosol is another crucial event in apoptosis, since the cytochrome c in the cytosol interacts with the protein Apaf-1 (apoptotic protease-activating factor-1), forming a complex capable of binding the protein pro-caspase 9 and activating it, by protein cleavage, to caspase 9. Caspase 9 has the ability to proteolytically activate executioner caspases 3 and 7, which are in turn responsible for the cleavage of parp-1 (poly ADP-ribose polymerase), an enzyme involved in the process of DNA repair and in maintaining genomic stability. The cleavage of parp-1 has the effect of inactivating it.

As demonstrated by the experiments illustrated in figures 3 and 4, therefore, the compounds for medical use of formula (A) according to the present invention are capable of initiating the apoptosis pathway. As mentioned previously, the best results in initiating the apoptotic mechanism were obtained for compound A4, which thus appears to be the most promising for the treatment of tumours, breast tumours in particular: compound A4 revealed to be capable of reducing the viability of tumour cells, but not of normal ones. In particular, as shown in detail in figures 3 and 4, said compound initiates an apoptotic mechanism in all three cell lines used, thereby inactivating the parp-1 protein (figure 3 A, B, C, which shows the presence of the cleaved form of parp-1, hence of its inactivation), decreasing the expression of the anti-apoptotic protein bcl-2 and causing the release of cytochrome c into the cytoplasm (figure 4B). The results of the reported experiments constitute a further confirmation of the action of the compounds of formula (A), and the compound of formula A4 in particular, on the mechanism of cell death by apoptosis.

The inactivation of parp-1 is an event of considerable importance as regards the treatment of tumours, above all breast tumours: molecules that inactivate parp-1 have been used in both *in vitro* and *in vivo* studies as a potential therapeutic strategy for the treatment of tumours with specific DNA repair defects, such as breast tumours of the "triple negative" type (TNBCs). Parp proteins are in fact critical for an appropriate repair of damage to DNA. The ability of the compounds of the present invention, compound A4 in particular, to inactivate the parp-1 protein thus represents a therapeutic advantage which makes said compounds extremely promising in therapy for tumours, breast tumours in particular, and above all those of the triple negative type, which presently have a very poor prognosis.

Another aspect of the invention relates to a pharmaceutical composition comprising one or more compounds for use as a medicament having the formula (I), as described herein, or pharmaceutically acceptable salts thereof. Said composition can have application in the medical field, in particular in the treatment of tumours and, specifically, of breast tumours. The pharmaceutical composition of the invention can be formulated with suitable excipients known to the person skilled in the art. For example, the composition can be formulated in liquid form or in solid form.

In another aspect, the present invention relates to a process for preparing the 6,6*a*-dihydrofuro[3,2-*b*]furan-2-(5H)one derivatives for use having the formula (A) as described here from 4-in-1,3-diol organic substrates having the following structure (I): 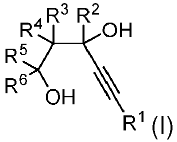 wherein
R¹ is selected from the group consisting of: H, alkyl, aryl, heteroaryl and trimethylsilyl; and
R², R³, R⁴, R⁵ and R⁶ are independently selected from the group consisting of: H, alkyl, aryl and heteroaryl.

In the process according to the invention, a catalytic double cyclisation reaction takes place, with the incorporation of a molecule of carbon monoxide (carbonylation) under oxidative conditions. The reaction takes place in a single stage and leads to the formation of the 6,6*a-*dihydrofuro[3,2-*b*]furan-2-(5*H*)one bicyclic derivatives of formula (A) according to the invention, generating only water as a co-product. The process of the invention is represented in the following reaction diagram 1. Diagram 1:

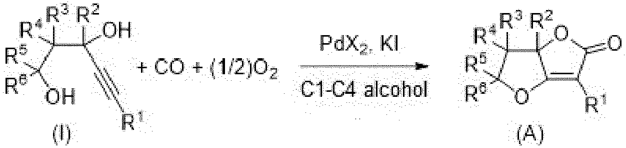

The process is conducted by reacting the 4-in-1,3-diol substrates with carbon monoxide (CO) and oxygen (O₂). The reaction takes place in the presence of a palladium halide (PdX₂, where X is a halogen) as a catalyst and potassium iodide (Kl) as an additive, in an alcoholic solvent.

In one embodiment, the palladium halide can be used in the process of the invention in a molar quantity comprised between 0.01 mol% and 10 mol% relative to the moles of the organic substrate (diol); preferably, the molar quantity can be comprised between 0.1 mol% and 10 mol%, and more preferably comprised between 1 mol% and 3 mol%, relative to the moles of the organic substrate (diol). In a particularly preferred embodiment, the palladium halide can be used in a molar quantity of about 2 mol% relative to the moles of the organic substrate (diol).

The palladium halide can preferably be palladium iodide (Pdl₂).

The potassium iodide (Kl) used as an additive in the reaction for synthesising the compounds of the invention has the function of solubilising and stabilising the palladium halide in the reaction mixture.

In one embodiment, the potassium iodide can be used in the process of the invention in a molar quantity comprised between 0.05 mol% and 100 mol% relative to the moles of the organic substrate (diol); the molar quantity can preferably be comprised between 0.5 mol% and 50 mol%, and more preferably comprised between 5 and 15 mol% relative to the moles of the organic substrate (diol). In a particularly preferred embodiment, the potassium iodide can be used in a molar quantity of about 10 mol% relative to the moles of the organic substrate (diol).

In one embodiment, the reaction can take place at a pressure comprised between 10 and 100 atmospheres; preferably, the reaction can take place at a pressure of about 40 atmospheres (± 2 atm). In a particularly preferred embodiment, the CO pressure can be about 32 atm (± 1 atm) and the air pressure can be about 8 atm (± 0.5 atm). The reaction typically takes place at a temperature comprised between 80°C and 120°C, preferably comprised between 90°C and 110°C, more preferably equal to about 100°C (± 2°C).

The duration of the reaction is generally comprised between 3 hours and 15 hours, depending on the reaction conditions used; the duration of the reaction can preferably be comprised between 6 hours and 12 hours.

The solvent used in the reaction is an alkyl alcohol having a linear or branched chain with a length comprised between 1 and 4 carbon atoms. For example, alcoholic solvents usable in the reaction can be methanol, ethanol, 1-propanol, 2-propanol, *n*-butanol, *sec*-butanol, isobutanol, *tert-*butanol and mixtures thereof. The solvent used can preferably be methanol.

The alcoholic solvent is used in an amount such that the concentration of the organic substrate (diol) in the solvent is comprised between 0.01 M and 0.5 M, preferably equal to about 0.05 M.

The process according to the invention is particularly advantageous, since it completes the synthesis of the compounds of formula (A) in a single step, using reagents that are commercially available or may be easily prepared in a laboratory and mild reaction conditions and generating only water as a reaction by-product. Therefore, the time, energy and costs necessary for carrying it out are modest.

Another aspect of the present invention relates to the compounds of formula (I), wherein R¹, R², R³, R⁴, R⁵ and R⁶ are defined as previously described, and wherein when R¹ is methyl or hydrogen, R² is selected from the group consisting of: aryl and heteroaryl.

In general, moreover, in said compounds R² can preferably be selected from the group consisting of alkyl, aryl and heteroaryl.

Other preferred embodiments of the compounds of formula (A) have been described with reference to the compounds of formula (A) for use as a medicament, and are to be considered valid, where applicable, also with reference to the compounds of formula (A) *per se.*

In another aspect, the invention is aimed at the use of the previously described 6,6*a*-dihydrofuro[3,2-*b*]furan-2-(5H)one derivatives of formula (A), or pharmaceutically acceptable salts thereof, in a therapeutic treatment method. Said therapeutic treatment method can preferably be applied on a human being.

In one embodiment of this aspect of the invention, the compounds of formula (A) or the pharmaceutically acceptable salts thereof can be used in a method for the therapeutic treatment of tumours; more particularly, they can be used in a method for the treatment of HR positive breast tumours, preferably ER+ and PR+ breast tumours. In another embodiment, the compounds of formula (A) or the pharmaceutically acceptable salts thereof can be used in a method for the treatment of breast tumours of the triple negative type, with represent the most aggressive form of cancer.

Some preferred embodiments of the compounds of formula (A) have been described with reference to the compounds of formula (A) for use as a medicament, and are to be considered valid, where applicable, also with reference to this aspect of the invention.

In the light of the foregoing, it has been ascertained in practice that the 6,6*a*-dihydrofuro[3,2-*b*]furan-2-(5H)one derivatives of formula (A) and the pharmaceutical compositions comprising them, according to the present invention, entirely fulfil the pre-established task, since they show a high antitumour activity (investigated in detail for compound A4), and in particular are capable of inducing cell death by apoptosis. Furthermore, the compounds of the invention are selective for tumour cells, as they have a low inhibitory effect against the viability of normal cells, with a low degree of overall toxicity and reduced side effects linked to their administration to human beings. These characteristics thus render the compounds and compositions of the invention effective in the medical field for the treatment of tumours, breast tumours in particular, since their effectiveness and low toxicity positively impact the quality of life and life expectancy of patients during the therapeutic treatment.

Furthermore, since the synthesis of the compounds of formula (A) by means of a catalytic double cyclisation with the incorporation of a molecule of carbon monoxide (carbonylation) as described here uses readily available starting reagents and mild reaction conditions, said compounds can be synthesized in a simple, rapid and economical manner.

Furthermore, it is to be understood that the characteristics of the embodiments described with reference to one aspect of the invention are to be considered valid also in relation to other aspects of the invention, even if not explicitly repeated.

### EXAMPLES

### Example 1: Synthesis of the 6,6a-dihydrofuro[3,2-b]furan-2-(5H)one derivatives

### Synthesis of compound A1

5.0 mg of Pdl₂ (1.39 □ 10⁻² mmol), 11.5 mg of Kl (6.93 □ 10⁻² mmol) and a 152.8 mg (0.7 mmol) solution of 2,4-dimethyl-6-phenyl-5-hexyne-2,4-diol in MeOH (14 ml) was loaded into a 250 ml stainless steel autoclave, in air. The autoclave was closed and, under stirring, pressurised with 32 atm of CO and 8 atm of air (up to a total of 40 atm). The reaction mixture was left under stirring at 100°C for 15 h. Finally, the autoclave was cooled, depressurised and opened. The solvent was removed by evaporation and the product 5,5,6*a*-trimethyl-3-phenyl-6,6*a*-dihydrofuro[3,2-*b*]furan-2(5*H*)-one(A1)was purified by means of a chromatography column on neutral alumina using a 9:1 hexane/ethyl acetate mixture as the eluent (yield: 56.4 mg, 33%).

White solid, melting point = 94.2-95.2°C.

IR (KBr): *v* = 2979 (m), 2929 (w), 1746 (s), 1667 (s), 1449 (m), 1388 (s), 1268 (m), 1185 (m), 1135 (m), 1090 (s), 1001 (w), 939 (s), 863 (w), 781 (m), 694 (s), 625 (w), 522 (w) cm⁻¹.

¹H NMR (300 MHz, CDCl₃): *δ* = 7.98-7.89 (m, 2H), 7.44-7.33 (m, 2H,), 7.31-7.21 (m, 2H,), 12.5 (d, *J =* 12.5, 1H), 2.10 (d, *J =* 12.5, 1H), 1.74 (s, 3H), 1.60 (s, 3H), 1.47 (s, 3H).

¹³C NMR (75 MHz, CDCl₃): *δ* = 182.0, 172.3,129.3, 128.4, 127.3, 126.8, 99.6, 99.2, 83.6, 45.4, 31.9, 28.1, 27.6.

GC-MS: *m*/*z* = 244 (23) [M⁺], 229 (1), 201 (2), 190 (4), 189 (31), 188 (62), 185 (14), 146 (11), 145 (100), 133 (4), 117 (11), 99 (1), 89 (27), 77 (3), 63 (6).

Elemental analysis: values calculated for C₁₅H₁₆O₃ (244.29): C, 73.75; H, 6.60; found: C, 73.79; H, 6.62.

### Synthesis of compound A2

5.0 mg of Pdl₂ (1.39 □ 10⁻² mmol), 11.5 mg of Kl (6.93 □ 10⁻² mmol) and a 150.1 mg (0.7 mmol) solution of 2,4-dimethyl-6-(trimethylsilyl)-5-hexyne-2,4-diol in MeOH (14 ml) was loaded, in air, into a 250 ml stainless steel autoclave. The autoclave was closed and, under stirring, pressurised with 32 atm of CO and 8 atm of air (up to a total of 40 atm). The reaction mixture was left under stirring at 100°C for 3 h. Finally, the autoclave was cooled, depressurised and opened. The solvent was removed by evaporation and the product 5,5,6*a*-trimethyl-3-(trimethylsilyl)-6,6*a-*dihydrofuro[3,2-*b*]furan-2(5*H*)-one (A2) was purified by means of a chromatography column on neutral alumina using a 9:1 hexane/ethyl acetate mixture as the eluent (yield: 154.6 mg, 92%).

White solid, melting point = 44.5-45.5°C.

IR (KBr): *v* = 2959 (m), 2900 (w), 1736 (s), 1638 (s), 1456 (m), 1381 (s), 1342 (s), 1268 (s), 1232 (s), 1123 (m), 1085 (m), 1006 (w), 961 (m), 917 (m), 848 (s), 778 (m), 699 (m), 622 (m), 532 (w) cm⁻¹.

¹H NMR (300 MHz, CDCl₃): δ =2.14 (d dist, *J* =12.5, 1H), 2.01 (d dist, *J* =12.5, 1H), 1.63 (s, 3H), 1.62 (s, 3H), 1.47 (s, 3H), 0.23 (s, 9H).

¹³C NMR (75 MHz, CDCl₃): *δ* = 195.4, 176.7, 97.4, 95.4, 85.1, 45.2, 31.4, 27.9, 27.4, -1.51.

GC-MS: *m*/*z* = 240 (12) [M⁺], 225 (15), 207 (20), 185 (14), 169 (6), 157 (10), 141 (42), 123 (3), 99 (29), 83 (5), 75 (23), 74 (9), 73 (100), 55 (6), 45 (13), 43 (41).

Elemental analysis: values calculated for C₁₂H₂₀O₃Si (240.37): C, 59.96; H, 8.39; Si, 11.68; found: C, 60.00; H, 8.37; Si, 11.71.

### Synthesis of compound A3

5.0 mg of Pdl₂ (1.39 □ 10⁻² mmol), 11.5 mg of Kl (6.93 □ 10⁻² mmol) and a 130.4 mg solution (0.7 mmol) of 3-methyl-5-(trimethylsilyl)-4-pentyne-1,3-diol in MeOH (14 ml) was loaded, in air, into a 250 ml stainless steel autoclave. The autoclave was closed and, under stirring, pressurised with 32 atm of CO and 8 atm of air (up to a total of 40 atm). The reaction mixture was left under stirring at 100°C for 3 h. Finally, the autoclave was cooled, depressurised and opened. The solvent was removed by evaporation and the product 6*a*-methyl-3-(trimethylsilyl)-6,6*a-*dihydrofuro[3,2-*b*]furan-2(5*H*)-one (A3) was purified by means of a chromatography column on neutral alumina using a 9:1 hexane/ethyl acetate mixture as the eluent (yield: 106.9 mg, 72%).

White solid, melting point 71.1-72.1 °C.

IR (KBr): *v* = 2981 (w), 2961 (m), 2934 (w), 2900 (w), 1741 (s), 1636 (s), 1446 (w), 1390 (m), 1302 (m), 1279 (s), 1238 (s), 1181 (m), 1141 (w), 1096 (m), 977 (m), 845 (s), 780 (m), 701 (w), 685 (w), 601 (w), 557 (w) cm⁻¹.

¹H NMR (500 MHz, CDCl₃): *δ* = 4.84-4.74 (m, 1H), 4.73-4.64 (m, 1H), 2.27-2.18 (m, 2H), 1.53 (s, 3H), 0.22 (s, 9H).

¹³C NMR (126 MHz, CDCl₃): *δ* = 198.2, 178.4, 95.3, 85.3, 36.3, 25.1, - 0.02.

GC-MS: *m*/*z* = 212 (7) [M⁺], 198 (15), 197 (100), 141 (14), 124 (6), 123 (72), 97(3), 75 (76), 74 (7), 73 (65), 69 (7), 45 (18), 43 (24).

Elemental analysis: values calculated for C₁₀H₁₆O₃Si (212.32): C, 56.57; H, 7.60; Si, 13.23; found: C, 56.61; H, 7.59; Si, 13.20.

### Synthesis of compound A4

5.0 mg of Pdl₂ (1.39 □ 10⁻² mmol), 11.5 mg of Kl (6.93 □ 10⁻² mmol) and a 193.5 mg solution of 2-methyl-4-phenyl-6-(trimethylsilyl)-5-hexyne-2,4-diol (0.7 mmol) in MeOH (14 ml) was loaded, in air, into a 250 ml stainless steel autoclave. The autoclave was closed and, under stirring, pressurised with 32 atm of CO and 8 atm of air (up to a total of 40 atm). The reaction mixture was left under stirring at 100°C for 3 h. Finally, the autoclave was cooled, depressurised and opened. The solvent was removed by evaporation and the product 5,5-dimethyl-6*a*-phenyl-3-(trimethylsilyl)-6,6*a-*dihydrofuro[3,2-*b*]furan-2(5*H*)-one (A4) was purified by means of a chromatography column on neutral alumina using a 9:1 hexane/ethyl acetate mixture as the eluent (yield: 190.3 mg, 90%).

White solid, melting point = 77.4-78.4 °C.

IR (KBr): *v* = 2956 (m), 2900 (w), 1741 (s), 1697 (s), 1495 (w), 1452 (m), 1390 (m), 1350 (s), 1268 (m), 1196 (m), 1128 (m), 1144 (m), 1055 (m), 987 (w), 937 (m), 847 (s), 755 (m), 704 (s), 629 (m), 580 (w) cm⁻¹.

¹H NMR (300 MHz, CDCl₃): δ = 7.36 (s, 5H), 2.73 (d, *J* =12.4, 1H), 2.30 (d, *J*=12.4, 1H), 1.52 (s, 3H), 1.11 (s, 3H), 0.30 (s, 9H).

¹³C NMR (75 MHz, CDCl₃): *δ* =191.1, 176.0, 140.5, 128.9, 126.5, 98.2, 97.7, 88.5, 46.2, 31.1, 26.7, -1.38.

GC-MS: *m*/*z* = 302 (18) [M⁺], 247 (7), 234 (9), 228 (2), 225 (1), 201 (9), 178 (16), 161 (20), 153 (1), 152 (1), 142 (9), 141 (67), 105 (45), 99 (2), 83 (2), 77 (21), 75 (16), 74 (9), 73 (100), 45 (12), 44 (9), 43 (6).

Elemental analysis: values calculated for C₁₇H₂₂O₃Si (302.45): C, 67.51; H, 7.33; Si, 9.29; found: C, 67.55; H, 7.34; Si, 9.31.

### Synthesis of compound A5

5.0 mg of Pdl₂ (1.39 □ 10⁻² mmol), 11.5 mg of Kl (6.93 □ 10⁻² mmol) and a 193.5 mg solution (0.7 mmol) of 2-methyl-4-phenyl-6-(trimethylsilyl)-5-hexyne-2,4-diol in MeOH (14 ml) was loaded, in air, into a 250 ml stainless steel autoclave. The autoclave was closed and, under stirring, pressurised with 32 atm of CO and 8 atm of air (up to a total of 40 atm). The reaction mixture was left under stirring at 100°C for 3 h. Finally, the autoclave was cooled, depressurised and opened. The solvent was removed by evaporation and the raw reaction product was diluted with 7 ml of tetrahydrofuran (THF). 201.3 mg (0.77 mmol) of tetrabutylammonium fluoride hydrate (TBAF nH₂O) was added to the solution and the reaction mixture was left under stirring at room temperature for 2 h. The solvent was removed by evaporation and the residue was diluted with 40 ml of ethyl ether and washed with 40 ml of H₂O. The aqueous phase was extracted with ethyl ether (3 x 40 ml) and the organic phases, joined together, were dried on anhydrous sodium sulphate. After filtration the solvent was removed by evaporation and the product 5,5-dimethyl-6*a-*phenyl-6,6*a*-dihydrofuro[3,2-*b*]furan-2(5*H*)-one (A5) was purified by means of a chromatography column on neutral alumina using a 9:1 hexane/ethyl acetate mixture as the eluent (yield: 127.2 mg, 79%).

White solid, melting point = 129.3-130.3 °C.

IR (KBr): *v* = 2980 (m), 2928 (w), 1766 (s), 1651 (s), 1451 (m), 1352 (m), 1267 (m), 1193 (m), 1128 (m), 1055 (m), 937 (m), 878 (m), 806 (m), 725 (w), 703 (s), 629 (w) cm⁻¹.

¹H NMR (300 MHz, CDCl₃): δ = 7.45-7.29 (m, 5H), 5.20 (s, 1H), 2.79 (d, *J*=12.4, 1H), 2.39 (d, *J*=12.4, 1H), 1.57 (s, 3H), 1.11 (s, 3H).

¹³C NMR (75 MHz, CDCl₃): *δ* =184.9, 173.6, 139.6, 129.2, 129.0, 126.4, 99.3, 89.0, 46.0, 31.1, 26.5.

GC-MS: *m*/*z* = 230 (5) [M⁺], 215 (30), 200 (7), 175 (13), 147 (6), 106 (8), 105 (100), 77 (27), 69 (11).

Elemental analysis: values calculated for C₁₄H₁₄O₃: C, 73.03; H, 6.13; found: C, 73.08; H, 6.14.

### Example 2: antitumour assays

### Cell cultures

All cell lines were obtained from the American Type Culture Collection (ATCC, Manassas, VA, USA). The human mammary adenocarcinoma cells (MCF-7) were cultured in DMEM-F12 (Dulbecco's Modified Eagle's Medium (DME) and Ham's F-12 Nutrient Mixture) containing 5% newborn calf serum (NCS), 1% glutamine, 1% penicillin/streptomycin (Sigma-Aldrich, Milan, Italy). The TNBC cells (MDA-MB-231 and MDA-MB-468) were cultured in DMEM-F12 containing 10% heat-inactivated foetal bovine serum (FBS), 1% glutamine, 1% penicillin/streptomycin (Sigma-Aldrich, Milan, Italy). The 3T3L- cells 1 (immortalised mouse fibroblasts) were cultured in DMEM (Dulbecco's Modified Eagle's medium) containing 10% heat-inactivated foetal bovine serum (FBS) and 1% penicillin/streptomycin (Sigma-Aldrich, Milan, Italy). The human mammary epithelial cells MCF-10A were maintained in DMEM-F12 with 5% horse serum (HS), 1% glutamine, 1% penicillin/streptomycin, 5 µg/ml of insulin, 0.25 mg/ml of hydrocortisone, 20 ng/ml of hEGF (human epidermal growth factor) and 0.05 mg/ml of cholera enterotoxin (Sigma-Aldrich, Milan, Italy).

All cell lines were maintained at 37°C in a 5% CO₂ humidified atmosphere and periodically screened for mycoplasma contamination.

### Cell viability assay: MTT test

Cell viability was evaluated using a colorimetric assay which exploits the conversion, by the mitochondrial enzyme succinate dehydrogenase (SDH), of the bromide reagent of 3-(4,5-dimethylthiazol-2-il)-2,5-diphenyltetrazole (MTT) (Sigma-Aldrich, Milan, Italy) into crystals of a product called formazan (blue-coloured). The reaction can take place only in metabolically active (and hence viable) cells and the absorbance value obtained with a spectrophotometric reading can be correlated to the number of viable cells present.

The cells were seeded in 48-well plates (25,000 cells/well), cultured for 72h in a complete medium before being treated for 72h with different doses (1, 5, 10, 20, 40 µM) of compounds A1, A2, A3, A4 and A5. At the end of the treatment the cells were incubated in darkness for 3h with 50 µl of a solution of MTT in PBS (2 mg/ml). The MTT solution was then carefully removed and the formazan salts that had formed were solubilised by adding 200 µl of DMSO (dimethyl sulphoxide) (Sigma-Aldrich, Milan, Italy), which imparts a pinkish colour to the solution. The coloured product was detected with the spectrophotometric method, by reading absorbance at the wavelength of 570 nm.

### Effect of the 6,6a-dihydrofuro[3,2-b]furan-2-(5H)one derivatives of the invention on the viability of MCF-7, MDA-MB-231 and MDA-MB-468 cells

With reference to figure 1, it may be observed that all of the tested compounds showed to be capable of reducing the viability of MCF-7, MDA-MB 231 and MDA-MB-468 cells. In particular, in the case of compound A1, a significant reduction in the viability of MCF-7 and MDA-MB231 cells was observed starting from a concentration of 10 µM, whereas MDA-MB-468 cells showed to be slightly more sensitive to this compound since a significant reduction in viability was obtained starting from a concentration of 5 µM (figure 1A). Compound A2 reduced the cell viability of all three cell lines starting from the lowest concentration among those tested (1 µM) (figure 1B). Compound A3 significantly influenced the cell viability of the TNBC (MDA-MB-231 and MDA-MB-468) cell lines starting from a concentration of 5 µM, whereas it was effective on MCF-7 cells starting from a concentration of 10 µM (figure 1C). Compound A4 reduced the viability of the cells MCF-7 (in the 1-40 µM range of concentrations), MDA-MB-231 and MDA-MB-468 (in the 10-40 µM range of concentrations) (figure 1D). Compound A5 caused a modest but significant effect on the viability of MCF-7 cells (in the 5-40 µM range of concentrations) and on MDA-MB-231 cells (in the 20-40 µM range of concentrations), whereas it was slightly more effective on MDA-MB-468 cells (in the 10-40 µM range of concentrations) (figure 1E).

Overall, these data indicate that, among all the cell lines examined, MDA-MD-468 seems to be the most sensitive to treatment with all of the tested compounds. This situation could be due to a different pattern of gene expression in this cell line: a clear definition of the target genes will contribute to explaining such a difference.

### Effect of the 6,6a-dihydrofuro[3,2-b]furan-2-(5H)one derivatives of the invention on the viability of MCF-10A and 3T3-L1 cells

Cell viability was evaluated with the MTT colorimetric assay as described above.

With reference to figure 2, it may be observed that the viability of MCF-1 0A and 3T3-L1 cells is not influenced by compounds A4 and A5 (figure 2D and 2E), whereas compounds A1, A2 and A3 (figure 2A, 2B and 2C) cause a reduction in cell viability in both cells types, modest in the case of compound A2, more substantial in the case of compounds A1 and A3. However, it should be observed that the reduction in cell viability of compounds A1 and A3 is obtained at rather high concentrations of the compound (in general 20-40 µM).

In particular, in MCF-10A cells, the derivative A1 exerts a slight but statistically significant inhibitory effect at the concentrations of 20 µM and 40 µM (figure 2A), whereas the inhibitory effect (in any case of modest entity) of A2 and of A3 is manifested, respectively, starting from a concentration of 5 µM (figure 2B) and 10 µM (figure 2C). In the 3T3-L1 cells, compounds A1 (figure 2A) and A3 (figure 2C) bring about an inhibitory effect only at the highest concentration among those tested (40 µM), whereas the inhibitory effect of A2 (figure 2B) initiates starting from a concentration of 20 µM.

These results suggest that, among all of the tested compounds, A4 brings about significant inhibitory effects on the cell viability of tumour cells, whereas its effect on the viability of normal cells is small or even absent.

### Effect of compound A4 of the invention on the induction of death by apoptosis: western blot analysis for determination of the cleavage/inactivation of the parp-1 protein in MCF7, MDA-MB231 and MDA-MB468 cells

The ability of the compound of formula A4 to induce the death of MCF-7, MDA-MB231 and MDA-MB468 tumour cells by apoptosis was evaluated by a western blot analysis aimed at determining the cleavage (and hence inactivation) of the parp-1 protein.

The MCF-7, MDA-MB-231 and MDA-MB-468 cells were cultured in a complete medium for 72h in 60 mm plates (1 x 10⁶ cells) before being treated for 48h with compound A4 (20 µM). After the treatment the cells were lysed in ice with RIPA (Radio-Immune Precipitation Assay) buffer (50 mM Tris HCl, pH 8.0, 150 mM NaCl, 1% NP-40, 0.5% sodium deoxycholate, 0.1% SDS, sodium dodecyl sulphate), containing protease inhibitors (1 mM phenylmethylsulphonyl fluoride (PMSF), 1mM sodium orthovanadate, 20 µM aprotinin, 20 µM leupeptin) for protein extraction. The lysates were maintained under ice for 10 minutes, then centrifuged for 10 minutes at 13000 rpm at 4°C. The protein content present in the supernatant was determined spectrophotometrically using the Bradford method. The proteins were separated, based on their molecular weight, in a sodium dodecyl sulphate-polyacrylamide gel (12%) (SDS-PAGE) and then transferred onto a nitrocellulose membrane. The membranes were incubated for 1 hour at room temperature with a blocking solution (5% skimmed milk powder in TBS-T 1X) in order to block the nonspecific binding sites; then they were washed three times with TBS-T 1X (TBST 10X: 100 mM Tris, 1 M NaCl, 1% Tween 20 (polyoxyethylenesorbitan monolaurate) and incubated overnight at 4°C with the following primary antibody (Ab): anti-parp-1 (1:1000) (Santa Cruz Biotechnology, Santa Cruz, CA, USA).

In order to be able to visualise the interaction between the primary antibody and the protein of interest, the membranes were incubated for 1 hour at room temperature, after washing with TBS-T 1X three times in order to remove any traces of the primary antibody, with a specific secondary antibody conjugated to the enzyme horseradish peroxidase (HRP) and directed against the constant portion of the primary antibody. Said enzyme catalyses, in the presence of appropriate substrates (luminol and hydrogen peroxide), a chemiluminescence reaction that brings about the generation of a luminous signal that can be impressed on a photographic plate (chemiluminescence method, ECL (enhanced chemiluminescence)). In order to normalise the result, the various membranes were hot stripped for 30 minutes at 50°C with a stripping buffer (62.5 mM Tris-HCl pH 6.7, 2% SDS, 100mM β-mercaptoethanol) and re-incubated overnight with the antibody anti-glyceraldehyde 3-phosphate dehydrogenase (GAPDH) (1: 1000) (Santa Cruz Biotechnology, Santa Cruz, CA, USA) used as a control for the quantities of proteins loaded.

The data shown in figure 3 (A, B, C) show that in both the MCF-7 cells (figure 3 A) and MDA-MB-231 cells (figure 3 B), as well as in the MDA-MB-468 cells (figure 3 C), which underwent treatment with compound A4, the cleaved form of the parp-1 protein may be seen.

### Effect of compound A4 of the invention on the induction of death by apoptosis: modulation of the expression of apoptotic markers

A further confirmation of the ability of the compound of formula A4 to induce the death, by apoptosis, of MCF-7, MDA-MB231 and MDA-MB468 tumour cells was given by the evaluation, through western blot analysis, of its effect on the expression of the anti-apoptotic bcl-2 protein and on the appearance of cytochrome c in the cytosolic compartment.

The cells were cultured in a complete medium for 72 h in 60 mm plates (1 x 10⁶ cells) before being treated for 48h with compound A4 (20 µM) in order to evaluate the protein expression of bcl-2 and the expression of the cytochrome c present in the cytosolic protein fraction.

For an analysis of the protein expression of bcl-2, after treatment the cells were lysed in ice with RIPA (Radio-Immune Precipitation Assay) buffer (50 mM Tris HCl, pH 8.0, 150 mM NaCl, 1% NP-40, 0.5% sodium deoxycholate, 0.1% SDS, sodium dodecyl sulphate), containing protease inhibitors (1 mM phenylmethylsulphonyl fluoride (PMSF), 1mM sodium orthovanadate, 20 µM aprotinin, 20 µM leupeptin) for protein extraction. The lysates were maintained under ice for 10 minutes, then centrifuged for 10 minutes at 13000 rpm at 4°C. The protein content present in the supernatant was determined spectrophotometrically using the Bradford method. The proteins were separated, based on their molecular weight, in a sodium dodecyl sulphate-polyacrylamide gel (12%) (SDS-PAGE) and then subjected to a western blot analysis as described above, using a specific primary anti-bcl-2 antibody (1:500) (Santa Cruz Biotechnology, Santa Cruz, CA, USA).

For the purpose of analysing the protein expression of the cytochrome c present in the cytosolic fraction, after treatment the cells were collected by centrifugation at 2500 rpm for 10 minutes at 4°C. The pellets were resuspended in 50 µl of sucrose buffer (250 mM sucrose, 10 mM Hepes, 10 mM KCI, 1.5 mM MgCl2, 1 mM EDTA, 1 mM EGTA) (Sigma-Aldrich, Milan, Italy) containing 20µM of aprotinin, 20 µM of leupeptin, 1mM of PMSF and 0.05% of digitonin (Sigma-Aldrich, Milan, Italy).

The cells were incubated for 20 minutes at 4°C and then centrifuged at 13000 rpm for 15 minutes at 4°C. The protein content present in the supernatant was determined spectrophotometrically using the Bradford method. The proteins were separated, based on their molecular weight, in a sodium dodecyl sulphate-polyacrylamide gel (15%) (SDS-PAGE) and then subjected to a western blot analysis as described above, using a specific primary anti-cytochrome c antibody (1:1000) (Santa Cruz Biotechnology, Santa Cruz, CA, USA).

With reference to figure 4, it may be observed that the treatment with compound A4 was capable of decreasing the expression of bcl-2 (Figure 4A) in all three breast cancer lines tested. The transfer of cytochrome c from mitochondria to the cytosol is a fundamental event for activating the mitochondria-dependent apoptotic pathway. As shown in figure 4B, the level of cytochrome c in the cytosolic fraction following treatment with compound A4 increased to a significant degree both in MCF-7 cells and in MDA-MB-231 and MDA-MB-468 cells. As a control for the amounts of proteins loaded, the membranes were incubated overnight with an anti-glyceraldehyde 3-phosphate dehydrogenase antibody (GAPDH) (1: 1000) (Santa Cruz Biotechnology, Santa Cruz, CA, USA).

## Claims

1. A compound having a formula of structure (A): 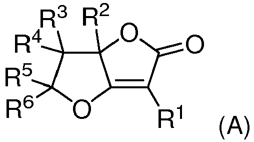 wherein:
R¹ is selected from the group consisting of: H, alkyl, aryl, heteroaryl and
trimethylsilyl;
R², R³, R⁴, R⁵ and R⁶ are independently selected from the group consisting of: H, alkyl, aryl and heteroaryl;
or a pharmaceutically acceptable salt thereof,
for use as a medicament.

2. The compound for use according to claim 1 or a pharmaceutically acceptable salt thereof, wherein R² is selected from the group consisting of alkyl, aryl and heteroaryl.

3. The compound for use according to claim 1 or 2, or a pharmaceutically acceptable salt thereof, wherein R² is phenyl.

4. The compound for use according to any one of the preceding claims, or a pharmaceutically acceptable salt thereof, wherein R¹ is trimethylsilyl.

5. The compound for use according to any one of the preceding claims, or a pharmaceutically acceptable salt thereof, wherein R³ and R⁴, independently of each other, are H.

6. The compound for use according to any one of the preceding claims, or a pharmaceutically acceptable salt thereof, wherein R⁵ and R⁶, independently of each other, are methyl.

7. The compound for use according to claim 1, selected from the group consisting of: 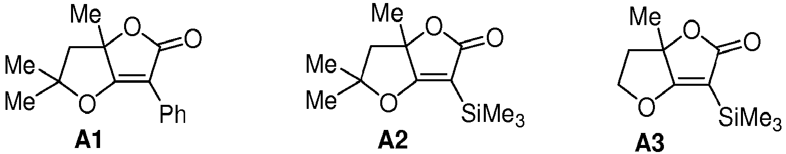 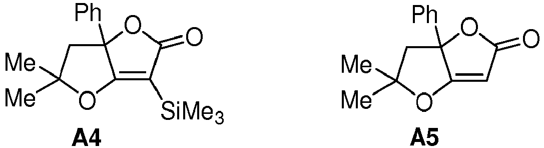 or a pharmaceutically acceptable salt thereof.

8. The compound for use according to any one of claims 1-7, or a pharmaceutically acceptable salt thereof, where said use is in the treatment of tumours.

9. The compound for use according to claim 8, or a pharmaceutically acceptable salt thereof, where said tumours are breast tumours.

10. A pharmaceutical composition comprising one or more compounds for use according to any one of claims 1-7, or pharmaceutically acceptable salts thereof.

11. A process for preparing the compound for use according to any one of claims 1-7, wherein a 4-in-1,3-diol compound having the structure (I) 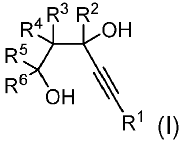 wherein
R¹ is selected from the group consisting of: H, alkyl, aryl, heteroaryl and trimethylsilyl;
R², R³, R⁴, R⁵ and R⁶ are independently selected from the group consisting of: H, alkyl, aryl and heteroaryl,
is reacted with carbon monoxide (CO) and oxygen (O₂) in the presence of a palladium halide (PdX₂, where X is a halogen) as a catalyst and potassium iodide (Kl) as additive, in a solvent comprising one or more alkyl alcohols having a linear or branched chain of a length comprised between 1 and 4 carbon atoms.

12. The process according to claim 11, wherein the palladium halide is palladium iodide (Pdl₂).

13. The process according to claim 11 or claim 12, wherein the solvent is methanol.

14. A compound having a formula of structure (A): 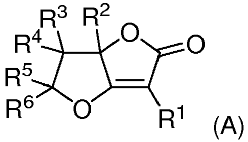 wherein:
R¹ is selected from the group consisting of: H, alkyl, aryl, heteroaryl and
trimethylsilyl;
R², R³, R⁴, R⁵ and R⁶ are independently selected from the group consisting of: H, alkyl, aryl and heteroaryl;
wherein when R¹ is methyl or hydrogen, R² is selected from the group consisting of: aryl and heteroaryl.

15. The compound according to claim 14, wherein R² is selected from the group consisting of alkyl, aryl and heteroaryl.

## Patentansprüche

1. Verbindung aufweisend eine Strukturformel (A): 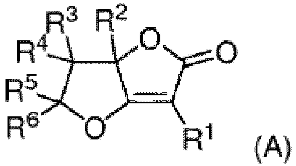 wobei:
R¹ aus der Gruppe ausgewählt ist, bestehend aus: H, Alkyl, Aryl, Heteroaryl und Trimethylsilyl;
R², R³, R⁴, R⁵ und R⁶ unabhängig aus der Gruppe ausgewählt sind, bestehend aus: H, Alkyl, Aryl und Heteroaryl;
oder ein pharmazeutisch annehmbares Salz davon zur Verwendung als Medikament.

2. Verbindung zur Verwendung nach Anspruch 1 oder ein pharmazeutisch annehmbares Salz davon, wobei R² aus der Gruppe ausgewählt ist, bestehend aus Alkyl, Aryl und Heteroaryl.

3. Verbindung zur Verwendung nach Anspruch 1 oder 2 oder ein pharmazeutisch annehmbares Salz davon, wobei R² Phenyl ist.

4. Verbindung zur Verwendung nach einem der vorhergehenden Ansprüche oder ein pharmazeutisch annehmbares Salz davon, wobei R¹ Trimethylsilyl ist.

5. Verbindung zur Verwendung nach einem der vorhergehenden Ansprüche oder ein pharmazeutisch annehmbares Salz davon, wobei R³ und R⁴, unabhängig voneinander, H sind.

6. Verbindung zur Verwendung nach einem der vorhergehenden Ansprüche oder ein pharmazeutisch annehmbares Salz davon, wobei R⁵ und R⁶, unabhängig voneinander, Methyl sind.

7. Verbindung zur Verwendung nach Anspruch 1, ausgewählt aus der Gruppe bestehend aus: 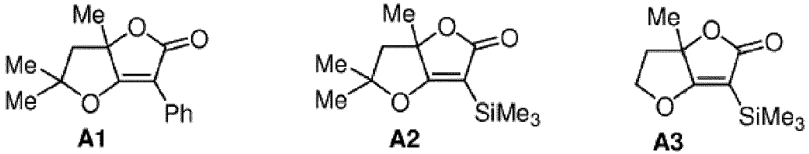 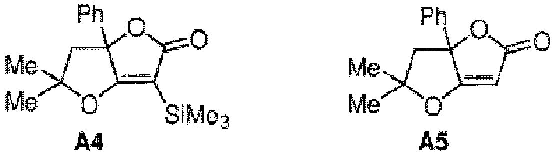 oder ein pharmazeutisch annehmbares Salz davon.

8. Verbindung zur Verwendung nach einem der Ansprüche 1-7 oder ein pharmazeutisch annehmbares Salz davon, wobei diese Verwendung die Behandlung von Krebs betrifft.

9. Verbindung zur Verwendung nach Anspruch 8 oder ein pharmazeutisch annehmbares Salz davon, wobei es sich bei Krebs um Brustkrebs handelt.

10. Pharmazeutische Zusammensetzung umfassend eine oder mehrere Verbindungen zur Verwendung nach einem der Ansprüche 1-7 oder pharmazeutisch annehmbare Salzen davon.

11. Verfahren zur Herstellung der Verbindung zur Verwendung nach einem der Ansprüche 1-7, wobei eine 4-in-1,3-Diolverbindung die Struktur (I) aufweist 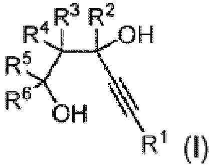 wobei
R¹ aus der Gruppe ausgewählt ist, bestehend aus: H, Alkyl, Aryl, Heteroaryl und Trimethylsilyl;
R², R³, R⁴, R⁵ und R⁶ unabhängig aus der Gruppe ausgewählt sind, bestehend aus: H, Alkyl, Aryl und Heteroaryl,
wird mit Kohlenmonoxid (CO) und Sauerstoff (02) in Gegenwart eines Palladiumhalogenids (PdX2, wobei X ein Halogen ist) als Katalysator und Kaliumiodid (KI) als Additiv in einem Lösungsmittel umgesetzt, umfassend einen oder mehrere Alkylalkohole mit einer linearen oder verzweigten Kette mit einer Länge zwischen 1 und 4 Kohlenstoffatomen.

12. Verfahren nach Anspruch 11, wobei das Palladiumhalogenid Palladiumiodid (PdI₂) ist.

13. Verfahren nach Anspruch 11 oder Anspruch 12, wobei es sich beim Lösungsmittel um Methanol handelt.

14. Verbindung aufweisend eine Strukturformel (A): 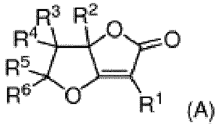 wobei:
R¹ aus der Gruppe ausgewählt ist, bestehend aus: H, Alkyl, Aryl, Heteroaryl und Trimethylsilyl;
R², R³, R⁴, R⁵ und R⁶ unabhängig aus der Gruppe ausgewählt sind, bestehend aus: H, Alkyl, Aryl und Heteroaryl;
wobei, wenn R¹ Methyl oder Wasserstoff ist, R² aus der Gruppe ausgewählt ist, bestehend aus: Aryl und Heteroaryl.

15. Verbindung nach Anspruch 14, wobei R² aus der Gruppe ausgewählt ist, bestehend aus Alkyl, Aryl und Heteroaryl.

## Revendications

1. Composé ayant une formule de structure (A) : 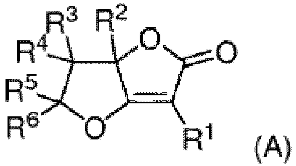 dans lequel :
R¹ est sélectionné dans le groupe constitué par : l'H, l'alkyle, l'aryle, l'hétéroaryle et le triméthylsilyle ;
R², R³, R⁴, R⁵ et R⁶ sont indépendamment choisis dans le groupe constitué par: l'H, l'alkyle, l'aryle et l'hétéroaryle ;
ou l'un de ses sels acceptables pharmaceutiquement, pour une utilisation comme médicament.

2. Composé pour son utilisation selon la revendication 1 ou l'un de ses sels acceptables pharmaceutiquement, dans lequel R² est choisi dans le groupe constitué par un alkyle, un aryle et un hétéroaryle.

3. Composé pour son utilisation selon la revendication 1 ou 2, ou l'un de ses sels pharmaceutiquement acceptables, dans lequel R² est un phényle.

4. Composé pour son utilisation selon l'une quelconque des revendications précédentes, ou l'un de ses sels pharmaceutiquement acceptables, dans lequel R¹ est un triméthylsilyle.

5. Composé pour son utilisation selon l'une quelconque des revendications précédentes, ou l'un de ses sels pharmaceutiquement acceptables, dans lequel R³ et R⁴, indépendamment l'un de l'autre, sont de l'H.

6. Composé pour son utilisation selon l'une quelconque des revendications précédentes, ou l'un de ses sels pharmaceutiquement acceptables, dans lequel R⁵ et R⁶, indépendamment l'un de l'autre, sont du méthyle.

7. Composé pour son utilisation selon la revendication 1, sélectionné dans le groupe constitué par : 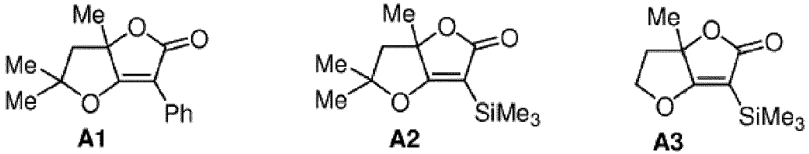 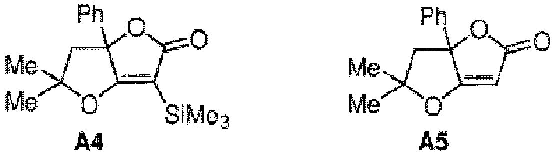 ou l'un de ses sels acceptables pharmaceutiquement.

8. Composé pour son utilisation selon l'une quelconque des revendications 1-7, ou l'un de ses sels pharmaceutiquement acceptables, où ladite utilisation sert au traitement des tumeurs.

9. Composé pour son utilisation selon la revendication 8, ou l'un de ses sels pharmaceutiquement acceptables, où lesdites tumeurs sont des tumeurs du sein.

10. Composition pharmaceutique comprenant un ou plusieurs composés pour une utilisation selon l'une quelconque des revendications 1-7, ou l'un de ses/leurs sels pharmaceutiquement acceptables.

11. Procédé de préparation du composé pour une utilisation selon l'une quelconque des revendications 1-7, dans lequel un composé 4-in-1,3-diol ayant la structure (1) 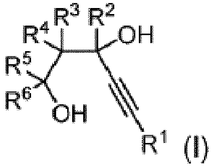 où
R¹ est sélectionné dans le groupe constitué par : l'H, l'alkyle, l'aryle, l'hétéroaryle et le triméthylsilyle ;
R², R³, R⁴, R⁵ et R⁶ sont indépendamment choisis dans le groupe constitué par: l'H, l'alkyle, l'aryle et l'hétéroaryle,
est mis à réagir avec du monoxyde de carbone (CO) et de l'oxygène (02) en présence d'un halogénure de palladium (PdX₂, où X est un halogène) comme catalyseur et d'iodure de potassium (KI) comme additif, dans un solvant comprenant un ou plusieurs alcools alkyliques comportant une chaîne linéaire ou ramifiée d'une longueur comprise entre 1 et 4 atomes de carbone.

12. Procédé selon la revendication 11, dans lequel l'halogénure de palladium est l'iodure de palladium (PdI₂) .

13. Procédé selon la revendication 11 ou la revendication 12, dans lequel le solvant est du méthanol.

14. Composé ayant une formule de structure (A) : 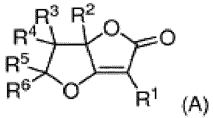 dans lequel :
R¹ est sélectionné dans le groupe constitué par : l'H, l'alkyle, l'aryle, l'hétéroaryle et le triméthylsilyle ;
R², R³, R⁴, R⁵ et R⁶ sont indépendamment choisis dans le groupe constitué par: l'H, l'alkyle, l'aryle et l'hétéroaryle ;
dans lequel, lorsque R¹ est du méthyle ou de l'hydrogène, R² est choisi dans le groupe constitué par : l'aryle et l'hétéroaryle.

15. Composé selon la revendication 14, dans lequel R² est choisi dans le groupe constitué de l'alkyle, l'aryle et l'hétéroaryle.
